# EUROPÄISCHE PATENTANMELDUNG

(11) **EP 3 231 859 A1**
(43) Veröffentlichungstag der Anmeldung: **18.10.2017**
(21) Anmeldenummer: 16000819.9
(22) Anmeldetag: 11.04.2016
(51) Int. Cl.: C12M 1/00

(54) **FÜR DIE KULTIVIERUNG PHOTOSYNTHETISCH AKTIVER ZELLEN GEEIGNETES REAKTIONSSYSTEM UND DESSEN VERWENDUNG**

(71) Anmelder: MIAL GmbH, 26160 Bad Zwischenhahn (DE)
(72) Erfinder: ÖLTJENBRUNS, Jörn, 26160 Bad Zwischenahn (DE)
(74) Vertreter: Bohmann, Armin K.

(57) **Zusammenfassung**

Die vorliegende Erfindung betrifft ein Reaktionssystem geeignet für die Kultivierung von photosynthetisch aktiven Zellen, umfassend in vertikaler Anordnung
- einen ersten Reaktionssystemteil, wobei der erste Reaktionssystemteil eine im Wesentlichen zylindrische Form aufweist, und
- einen zweiten Reaktionssystemteil, wobei der zweite Reaktionssystemteil eine im Wesentlichen kegelstumpfförmige Form aufweist,
wobei zumindest ein Teil der ersten Reaktionssystemteils und/oder ein Teil des zweiten Reaktionssystemteils Eintritt von Licht in das Innere des Reaktionssystems erlaubt und wobei die kegelstumpfförmige Form des zweiten Reaktionssystemteils einen Winkel α aufweist, der Winkel α der zwischen der Mantellinie des Kegelstumpfes und dem Radius der Grundfläche des Kegelstumpfes eingeschlossene Winkel ist, und der Winkel α einen Wert von 55° bis 67° aufweist.

## Beschreibung

Die vorliegende Erfindung betrifft ein Reaktionssystem, welches für die Kultivierung von photosynthetisch aktiven Zellen geeignet ist, eine Reaktionssystemanordnung sowie ein Verfahren für die Kultivierung von photosynthetisch aktiven Zellen.

Die Kultivierung von photosynthetisch aktiven Zellen und Algen im Besonderen ist die für die Erzeugung von Kraftstoffen, Biodiesel, Düngemittel, Farbstoffen und Pharmazeutika von Bedeutung. Die photosynthetisch aktiven Zellen können dabei grundsätzlich entweder in offenen Becken bzw. Kanälen oder in Photobioreaktoren kultiviert werden. Die Art der Kultivierung hängt typischerweise von dem Verwendungszweck der kultivierten Zellen ab. So werden photosynthetisch aktive Zellen vor allem dann in offenen Becken oder Gerinnen kultiviert, wenn die Gewinnung derselben in einem großen Maßstab erforderlich ist oder aber die Kultivierung der Zellen durch Kontaminationen für den angestrebten Verwendungszweck der kultivierten Zellen nicht in erheblichem Maße beeinträchtigt wird. Umgekehrt werden geschlossene Systeme und insbesondere Photobioreaktoren dann bevorzugt, wenn die Kultivierung ohne Kontamination durch Mikroorganismen erfolgen soll oder muss.

Photobioreaktoren umfassen zumindest ein Reaktionsgefäß, in dem die photosynthetisch aktiven Zellen kultiviert werden. Das Reaktionsgefäß muss gewährleisten, dass die im Reaktionsgefäß enthaltenen Zellen in dem Umfang dem Licht ausgesetzt sind, so dass diese Photosynthese durchführen können. Hierzu sind im Stand der Technik verschiedene Konzepte beschrieben. Besonders verbreitet sind Rührkesselreaktoren oder Airlift-Reaktoren, mit einer lichtdurchlässigen Außenwand. Diese Außenwand oder ein Teil davon kann bspw. aus Glas oder einer Membran oder Folie hergestellt sein.

Bei den Photobioreaktoren vom Rührkesseltyp erfolgt die Durchmischung des Inhaltes des Reaktionsgefäßes durch ein im Reaktionsgefäß angebrachtes Rührwerk. Durch das Rührwerk wird Energie in das Reaktionsgefäß eingetragen, die eine Durchmischung oder Umwälzung des Inhaltes des Reaktionsgefäßes bedingt. Diese Durchmischung hat zur Folge, dass die im Reaktionsgefäß enthaltenen photosynthetisch aktiven Zellen dem in der Regel von außen, durch eine Wand des Reaktionsgefäßes eingestrahlten Licht ausgesetzt werden, das ohne diese Durchmischung lediglich jene Zellen erreichen würde, die sich nahe an der Wand des Reaktionsgefäßes auflialten, wodurch die Menge an tatsächlich Photosynthese betreibenden photosynthetisch aktiven Zellen im Reaktionsgefäß beschränkt wäre. Des Weiteren führt die Durchmischung auch dazu, dass Transportprozesse zwischen den photosynthetisch aktiven Zellen und dem im Reaktionsgefäß enthaltenen Kultivierungsmedium dadurch beschleunigt werden, dass die Ausbildung von Grenzschichten zwischen den Zellen und dem Kultivierungsmedium verhindert oder zumindest verringert wird. Schließlich verhindert die Durchmischung das Absinken der Zellen, wodurch diese sowohl aus dem Bereich der wirksamen Einstrahlung von Licht als auch aus dem Bereich entfernt würden, in dem die für die Kultivierung erforderlichen Gase und Nährstoffe vorliegen, was wiederum einer Verringerung der im Reaktionsgefäß enthaltenen Masse an tatsächlich photosynthetisch aktiven Zellen zur Folge hätte.

Bei Airlift-Reaktoren liegt im Gegensatz zu Reaktionsgefäßen vom Rührkesseltyp kein mechanisches Rührwerk vor. Stattdessen wird die Umwälzung durch einen kontrollierten Gaseintrag erreicht, der am oder durch den Boden des Reaktionsgefäßes erfolgt. Das eingetragene Gas tritt an der Kopfseite des Reaktionsgefäßes wieder aus. Durch die dadurch entstehende hydrostatische Druckdifferenz ergibt sich eine Pumpenwirkung, die einen Flüssigkeitsstrom aus dem im Reaktionsgefäß enthaltenen Kultivierungsmedium von unten nach oben bewirkt, wobei am Kopfende des Reaktionsgefäßes eine Rückströmung entsteht, die das gaslose Kultivierungsmedium wieder zum Boden des Reaktionsgefäßes befördert. Die Prozesse, die durch die Umwälzung in Airlift-Reaktoren stattfinden bzw. ausgelöst werden, sind grundsätzlich identisch mit jenen von Reaktionsgefäßen vom Rührkesseltyp, wenngleich typischerweise bei Airlift-Reaktoren die eingetragene Energiemenge geringer ist als bei Reaktionsgefäßen vom Rührkesseltyp.

Der vorliegenden Erfindung liegt die Aufgabe zu Grunde, ein Reaktionssystem für die Kultivierung von photosynthetisch aktiven Zellen bereitzustellen. Insbesondere liegt der vorliegenden Erfindung die Aufgabe zu Grunde, ein Reaktionssystem für die Kultivierung von photosynthetisch aktiven Zellen bereitzustellen, welches leicht skalierbar ist. Des Weiteren liegt der vorliegenden Erfindung die Aufgabe zu Grunde, ein Reaktionssystem für die Kultivierung von photosynthetisch aktiven Zellen bereitzustellen, welches eine Durchmischung der in einem Kultivierungsmedium enthaltenen photosynthetisch aktiven Zellen bei geringem Energieeintrag erlaubt; bevorzugterweise wirkt sich der geringe Energieeintrag nicht nachteilig auf das Kultivierungsergebnis aus, wobei das Kultivierungsergebnis bevorzugtererweise die durch die Kultivierung der photosyntethisch aktiven Zellen erhaltene Biomasse an photosynthetisch aktiven Zellen oder ein durch die photosynthetisch aktiven Zellen erzeugtes Produkt ist.

Diese und weitere der vorliegenden Erfindung zu Grunde liegenden Aufgaben werden durch den Gegenstand der beigefügten unabhängigen Ansprüche gelöst. Bevorzugte Ausführungsformen sind Gegenstand der beigefügten abhängigen Ansprüche.

Genauer werden die der vorliegenden Erfindung zu Grunde liegenden Aufgaben auch gelöst durch den Gegenstand der folgenden Ausführungsformen 1 bis 63.
Ausführungsform 1. Reaktionssystem geeignet für die Kultivierung von photosynthetisch aktiven Zellen, umfassend in vertikaler Anordnung
   - einen ersten Reaktionssystemteil, wobei der erste Reaktionssystemteil eine im Wesentlichen zylindrische Form aufweist, und
   - einen zweiten Reaktionssystemteil, wobei der zweite Reaktionssystemteil eine im Wesentlichen kegelstumpfförmige Form aufweist,
   wobei zumindest ein Teil des ersten Reaktionssystemteils und/oder ein Teil des zweiten Reaktionssystemteils Eintritt von Licht in das Innere des Reaktionssystems erlaubt.
Ausführungsform 2. Reaktionssystem nach Ausführungsform 1, wobei die kegelstumpfförmige Form des zweiten Reaktionssystemteils einen Winkel α aufweist, der Winkel α der Winkel zwischen der Mantellinie des Kegelstumpfes und dem Radius der Grundfläche des Kegelstumpfes eingeschlossene Winkel ist, und der Winkel α so ausgewählt ist, dass photosynthetisch aktive Zellen auf der durch die Mantellinie definierten Mantelfläche zur Deckfläche des Kegelstumpfes sinken.
Ausführungsform 3. Reaktionssystem nach einer der Ausführungsformen 1 bis 2, wobei der Winkel α so ausgewählt ist, dass die photosynthetisch aktiven Zellen auf der durch die Mantellinie definierten Mantelfläche zur Deckfläche des Kegelstumpfes unter dem Einfluss einer im Reaktionssystem induzierten Strömung einer im Reaktionssystem enthaltenen Flüssigkeit sinken.
Ausführungsform 4. Reaktionssystem nach einer der Ausführungsformen 1 bis 3, wobei der Winkel α einen Wert von 55° bis 67° aufweist.
Ausführungsform 5. Reaktionssystem nach einer der Ausführungsformen 1 bis 4, wobei der Winkel α einen Wert von 60° bis 65° aufweist.
Ausführungsform 6. Reaktionssystem nach einer der Ausführungsformen 1 bis 5, wobei der Winkel α einen Wert von 65°aufweist.
Ausführungsform 7. Reaktionssystem nach einer der Ausführungsformen 1 bis 6, wobei der erste Reaktionssystemteil in Vertikalrichtung eine Höhe von 50 cm bis 135 cm aufweist.
Ausführungsform 8. Reaktionssystem nach einer der Ausführungsformen 1 bis 7, wobei der erste Reaktionssystemteil in Vertikalrichtung eine Höhe von 120 cm bis 135 cm aufweist.
Ausführungsform 9. Reaktionssystem nach einer der Ausführungsformen 1 bis 8, wobei der erste Reaktionssystemteil in Vertikalrichtung eine Höhe von 125 cm bis 130 cm aufweist.
Ausführungsform 10. Reaktionssystem nach einer der Ausführungsformen 1 bis 9, wobei der erste Reaktionssystemteil in Vertikalrichtung eine Höhe von 130 cm aufweist.
Ausführungsform 11. Reaktionssystem nach einer der Ausführungsformen 1 bis 10, wobei der Radius der zylindrischen Form des ersten Reaktionssystemteils einen Wert von 7,5 cm bis 40 cm aufweist.
Ausführungsform 12. Reaktionssystem nach einer der Ausführungsformen 1 bis 11, wobei der Radius der zylindrischen Form des ersten Reaktionssystemteils einen Wert von 10 cm bis 17,5 cm aufweist.
Ausführungsform 13. Reaktionssystem nach einer der Ausführungsformen 1 bis 12, wobei der Radius der zylindrischen Form des ersten Reaktionssystemteils einen Wert von 15 cm bis 16 cm aufweist.
Ausführungsform 14. Reaktionssystem nach einer der Ausführungsformen 1 bis 13, wobei der Radius der zylindrischen Form des ersten Reaktionssystemteils einen Wert von 15,5 cm aufweist.
Ausführungsform 15. Reaktionssystem nach einer der Ausführungsformen 1 bis 14, wobei die Höhe des ersten Reaktionssystemteils in Vertikalrichtung 130 cm beträgt, der Radius der zylindrischen Form des ersten Reaktionssystemteils 15,5 cm beträgt und die kegelstumpfförmige Form des zweiten Reaktionssystemteils ein Kegel ist, wobei die Höhe der kegelstumpfförmigen Form des zweiten Reaktionssystemteils 34 cm beträgt.
Ausführungsform 16. Reaktionssystem nach einer der Ausführungsformen 1 bis 15, wobei das Reaktionssystem eine Begasungseinrichtung zum Einführen von Gas in das Reaktionssystem umfasst.
Ausführungsform 17. Reaktionssystem nach Ausführungsform 15, wobei die Begasungseinrichtung das Gas im Bereich der Deckfläche des zweiten Reaktionssystemteils mit im Wesentlichen kegelstumpfförmiger Form einführt.
Ausführungsform 18. Reaktionssystem nach Ausführungsform 15, wobei der zweite Reaktionssystemteil eine kegelförmige Form aufweist und die Begasungseinrichtung das Gas im Bereich der Spitze des zweiten Reaktionssystemteils mit im Wesentlichen kegelförmiger Form einführt.
Ausführungsform 19. Reaktionssystem nach einer der Ausführungsformen 16 bis 18, wobei das Gas ein Trägergas und CO₂ umfasst.
Ausführungsform 20. Reaktionssystem nach Ausführungsform 19, wobei das Trägergas ausgewählt ist aus der Gruppe umfassend Luft und Stickstoff.
Ausführungsform 21. Reaktionssystem nach einer der Ausführungsformen 16 bis 20, wobei die Begasungseinrichtung eine solche ist, die eine Blasensäule erzeugen kann.
Ausführungsform 22. Reaktionssystem nach einer der Ausführungsform 16 bis 21, wobei die Blasen der Blasensäule einen Durchmesser von 0,05 cm bis 5 cm aufweisen.
Ausführungsform 23. Reaktionssystem nach einer der Ausführungsformen 16 bis 22, wobei die Blasen der Blasensäule einen Durchmesser von 0,2 cm bis 3 cm aufweisen.
Ausführungsform 24. Reaktionssystem nach einer der Ausführungsform 16 bis 23, wobei die Blasen der Blasensäule einen Durchmesser von 0,5 cm bis 2 cm aufweisen.
Ausführungsform 25. Reaktionssystem nach einer der Ausführungsformen 16 bis 24, wobei ein durch die Begasungseinrichtung in das Reaktionssystem eingebrachte Volumenstrom 10 ml/min pro Liter in dem Reaktionssystem enthaltener Flüssigkeit bis 45 ml/min pro Liter in dem Reaktionssystem enthaltener Flüssigkeit beträgt.
Ausführungsform 26. Reaktionssystem nach einer der Ausführungsformen 16 bis 25, wobei ein durch die Begasungseinrichtung in das Reaktionssystem eingebrachte Volumenstrom 12 ml/min pro Liter in dem Reaktionssystem enthaltener Flüssigkeit bis 20 ml/min pro Liter in dem Reaktionssystem enthaltener Flüssigkeit beträgt.
Ausführungsform 27. Reaktionssystem nach einer der Ausführungsformen 16 bis 26, wobei ein durch die Begasungseinrichtung in das Reaktionssystem eingebrachte Volumenstrom 13 ml/min pro Liter in dem Reaktionssystem enthaltener Flüssigkeit bis 16 ml/min pro Liter in dem Reaktionssystem enthaltener Flüssigkeit beträgt.
Ausführungsform 28. Reaktionssystem nach einer der Ausführungsformen 16 bis 27, wobei ein durch die Begasungseinrichtung in das Reaktionssystem eingebrachte Volumenstrom 13,3 ml/min pro Liter in dem Reaktionssystem enthaltener Flüssigkeit beträgt. Ausführungsform 29. Reaktionssystem nach einer der Ausführungsformen 1 bis 28, wobei das Reaktionssystem einen Kopfbereich umfasst, der sich, in Vertikalrichtung, an das obere Ende des ersten Reaktionssystemteils anschließt.
Ausführungsform 30. Reaktionssystem nach Ausführungsform 29, wobei der Kopfbereich, in Vertikalrichtung, den ersten Reaktionssystemteil nach oben abschließt und verschließt
Ausführungsform 31. Reaktionssystem nach einer der Ausführungsformen 29 bis 30, wobei der Kopfbereich mindestens einen Gasauslass umfasst.
Ausführungsform 32. Reaktionssystem nach Ausführungsform 31, wobei der Gasauslass ein Loch in dem eine Außenwand des Kopfbereiches des den ersten Reaktionssystem ausbildenden Materials ist.
Ausführungsform 33. Reaktionssystem nach einer der Ausführungsformen 29 bis 32, wobei der erste Reaktionssystemteil und der Kopfbereich zusammen einstückig ausgebildet sind.
Ausführungsform 33. Reaktionssystem nach einer der Ausführungsformen 1 bis 32, wobei der erste Reaktionssystemteil und der zweite Reaktionssystemteil miteinander verbunden sind.
Ausführungsform 34. Reaktionssystem nach einer der Ausführungsformen 1 bis 33, wobei der erste Reaktionssystemteil und der zweite Reaktionssystemteil zusammen einstückig ausgebildet sind.
Ausführungsform 35. Reaktionssystem nach einer der Ausführungsformen 1 bis 34, wobei der erste Reaktionssystemteil aus einem Material besteht, das ausgewählt ist aus der Gruppe umfassend lichtdurchlässige Kunststofffolie und Glas.
Ausführungsform 36. Reaktionssystem nach einer der Ausführungsformen 1 bis 35, wobei der zweite Reaktionssystemteil aus einem Material besteht, das ausgewählt ist aus der Gruppe umfassend lichtdurchlässige Kunststofffolie und Glas.
Ausführungsform 37. Reaktionssystem nach einer der Ausführungsformen 1 bis 36, wobei der Kopfbereich des ersten Reaktionssystemteils aus einem Material besteht, das ausgewählt ist aus der Gruppe umfassend licht durchlässige Kunststofffolie und Glas.
Ausführungsform 38. Reaktionssystem nach einer der Ausführungsformen 1 bis 37, wobei der erste Reaktionssystemteil und der Kopfbereich aus demselben Material bestehen.
Ausführungsform 39. Reaktionssystem nach einer der Ausführungsformen 1 bis 38, wobei der erste Reaktionssystemteil und der zweite Reaktionssystemteil aus demselben Material bestehen.
Ausführungsform 40. Reaktionssystem nach einer der Ausführungsformen 1 bis 39, wobei die photosynthetisch aktiven Zellen ausgewählt sind aus der Gruppe umfassend Pflanzenzellen und Algenzellen.
Ausführungsform 41. Reaktionssystem nach einer der Ausführungsformen 1 bis 40, wobei die photosynthetisch aktiven Zellen ausgewählt sind aus der Gruppe umfassend Chlorella, Nannochloropsis, Scenedescmus, Porphyridium, Spirulina, Chlamydomonas und Oedogonium.
Ausführungsform 42. Reaktionssystem nach einer der Ausführungsformen 1 bis 41, wobei die photosynthetisch aktiven Zellen ausgewählt sind aus der Gruppe umfassend Chlorella vulgaris, Chlorella kessleri und Chlorella sorokiniana.
Ausführungsform 43. Reaktionssystem nach einer der Ausführungsformen 1 bis 42, wobei das Reaktionssystem photosynthetisch aktive Zellen enthält und wobei die photosynthetisch aktiven Zellen ausgewählt sind aus der Gruppe umfassend Pflanzenzellen und Algenzellen.
Ausführungsform 44. Reaktionssystem nach einer der Ausführungsformen 1 bis 42, wobei das Reaktionssystem photosynthetisch aktive Zellen enthält und wobei die photosynthetisch aktiven Zellen ausgewählt sind aus der Gruppe umfassend Chlorella, Nannochloropsis, Scenedescmus, Porphyridium, Spirulina, Chlamydomonas und Oedogonium.
Ausführungsform 45. Reaktionssystem nach einer der Ausführungsformen 1 bis 42, wobei wobei das Reaktionssystem photosynthetisch aktive Zellen enthält und die photosynthetisch aktiven Zellen ausgewählt sind aus der Gruppe umfassend Chlorella vulgaris, Chlorella kessleri und Chlorella sorokiniana.
Ausführungsform 46. Reaktionssystemanordnung umfassend mindestens zwei Reaktionssysteme nach einer der Ausführungsformen 1 bis 45.
Ausführungsform 47. Verfahren zur Kultivierung von photosynthetisch aktiven Zellen umfassend.
   - Kultivieren von photosynthetisch aktiven Zellen in einem Reaktionssystem nach einer der Ausführungsformen 1 bis 45 oder einer Reaktionssystemanordnung nach Anspruch 46.
Ausführungsform 48. Verfahren nach Ausführungsform 47, wobei sich das Reaktionssystem oder die Reaktionssystemanordnung in einem Gewächshaus befindet.
Ausführungsform 49. Verfahren nach Ausführungsform 48, wobei die Temperierung des Reaktionssystems oder der Reaktionssystemanordnung über eine Temperierung des Gewächshauses erfolgt.
Ausführungsform 50. Verfahren nach einer der Ausführungsformen 47 bis 49, wobei das für die Photosynthese der photosynthetisch aktiven Zellen erforderliche Licht durch Sonnenlicht bereitgestellt wird.
Ausführungsform 51. Verfahren nach einer der Ausführungsformen 47 bis 50, wobei das für die Photosynthese der photosynthetisch aktiven Zellen erforderlich Licht nicht durch eine künstliche Lichtquelle bereitgestellt wird.
Ausführungsform 52. Verfahren nach einer der Ausführungsformen 47 bis 51, wobei das Gas ein Trägergas und CO₂ umfasst.
Ausführungsform 53. Verfahren nach Ausführungsform 52, wobei das Trägergas ausgewählt ist aus der Gruppe umfassend Luft und Stickstoff.
Ausführungsform 54. Verfahren nach einer der Ausführungsformen 52 bis 53, wobei das CO₂ der Einstellung des pH-Wertes der in dem Reaktionssystem enthaltenen Flüssigkeit dient.
Ausführungsform 55. Verfahren nach einer der Ausführungsformen 47 bis 54, wobei das Gas in Form von Blasen in das Reaktionssystem eingebracht wird, wobei die Blasen der Blasensäule einen Durchmesser von 0,05 cm bis 5 cm aufweisen.
Ausführungsform 56. Verfahren nach Ausführungsform 55, wobei die Blasen der Blasensäule einen Durchmesser von 0,2 cm bis 3 cm aufweisen.
Ausführungsform 57. Verfahren nach einer der Ausführungsformen 55 bis 56, wobei die Blasen der Blasensäule einen Durchmesser von 0,5 cm bis 2 cm aufweisen.
Ausführungsform 58. Verfahren nach einer der Ausführungsformen 47 bis 57, wobei ein durch die Begasungseinrichtung in das Reaktionssystem eingebrachter Volumenstrom 10 ml/min pro Liter in dem Reaktionssystem enthaltener Flüssigkeit bis 45 ml/min pro Liter in dem Reaktionssystem enthaltener Flüssigkeit beträgt.
Ausführungsform 59. Verfahren nach einer der Ausführungsformen 47 bis 58, wobei ein durch die Begasungseinrichtung in das Reaktionssystem eingebrachte Volumenstrom 12 ml/min pro Liter in dem Reaktionssystem enthaltener Flüssigkeit bis 20 ml/min pro Liter in dem Reaktionssystem enthaltener Flüssigkeit beträgt.
Ausführungsform 60. Verfahren nach einer der Ausführungsformen 47 bis 59, wobei ein durch die Begasungseinrichtung in das Reaktionssystem eingebrachte Volumenstrom 13 ml/min pro Liter in dem Reaktionssystem enthaltener Flüssigkeit bis 16 ml/min pro Liter in dem Reaktionssystem enthaltener Flüssigkeit beträgt.
Ausführungsform 61. Verfahren nach einer der Ausführungsformen 47 bis 60, wobei ein durch die Begasungseinrichtung in das Reaktionssystem eingebrachte Volumenstrom 13,3 ml/min pro Liter in dem Reaktionssystem enthaltener Flüssigkeit beträgt.
Ausführungsform 62. Verfahren nach einer der Ausführungsformen 47 bis 61, wobei die photosynthetisch aktiven Zellen ausgewählt sind aus der Gruppe umfassend Pflanzenzellen und Algenzellen.
Ausführungsform 63. Verfahren nach einem der Ausführungsformen 47 bis 62, wobei die photosynthetisch aktiven Zellen ausgewählt sind aus der Gruppe umfassend Chlorella, Nannochloropsis, Scenedescmus, Porphyridium, Spirulina, Chlamydomonas und Oedogonium.
Ausführungsform 63. Verfahren nach einer der Ausführungsformen 47 bis 63, wobei die photosynthetisch aktiven Zellen ausgewählt sind aus der Gruppe umfassend Chlorella vulgaris, Chlorella kessleri und Chlorella sorokiniana.

Der vorliegenden Erfindung liegt die überraschende Erkenntnis zugrunde, dass durch die Ausgestaltung des erfindungsgemäßen Reaktionssystem, welches für die Kultivierung von photosynthetisch aktiven Zellen geeignet ist, wobei das Reaktionssystem, in vertikaler Anordnung, einen ersten Reaktionssystemteil und daran anschließend einen zweiten Reaktionssystemteil der Gestalt umfasst, dass der erste Reaktionssystemteil eine im Wesentlichen zylindrische Form aufweist und der zweite Reaktionssystemteil eine im Wesentlichen kegelstumpfförmige Form aufweist, eine für die Kultivierung der photosynthetisch aktiven Zellen im Reaktionssystem ausreichende Durchmischung bei vergleichsweise geringem Energieeintrag gewährleistet werden kann. Bevorzugterweise erfolgt der Energieeintrag durch Einführen von Gas in den zweiten Reaktionssystemteil des erfindungsgemäßen Reaktionssystems. Genauer liegt der vorliegenden Erfindung die überraschende Erkenntnis zu Grunde, dass durch die Ausgestaltung der kegelstumpfförmigen Form des zweiten Reaktionssystemteils und insbesondere der Schrägung der kegelstumpfförmigen Form ein Absinken der photosynthetisch aktiven Zellen im Reaktionssystem im Sinne eines Abrutschens der photosynthetisch aktiven Zellen so erfolgt, dass sich diese auf der Deckfläche des Kegelstumpfes sammeln, von wo aus sie unmittelbar wieder in das Reaktionssystem und insbesondere in die im Reaktionssystem enthaltene Flüssigkeit zurückgeführt werden, ohne dass die Zellen sich auf der Deckfläche länger befinden, was zu einer Beeinträchtigung des Wachstums und/oder des Produktionsverhaltens der photosynthetisch aktiven Zellen führen würde. Die Rückführung der photosynthetischen Zellen von der Deckfläche des Kegelstumpfes erfolgt durch die durch das Einführen von Gas bedingte Flüssigkeitsströmung im Reaktionssystem oder direkt durch das Einführen von Gas in das Reaktionssystem und in den zweiten, unteren Reaktionssystemteil im Speziellen.

Es ist im Rahmen der vorliegenden Erfindung, dass die kegelstumpfförmige Form des zweiten Reaktionssystemteils als Kegel ausgeführt ist. In dieser Ausführungsform ist die Deckfläche der kegelstumpfförmigen Form die Spitze des Kegels, wobei die hierin im Zusammenhang mit der Deckfläche der kegelstumpfförmigen Form des zweiten Reaktionssystemteils offenbarten und beschriebenen Vorgänge im Wesentlichen in der Spitze des Kegels des zweiten Reaktionssystemteils ablaufen oder ablaufen können.

Zur Beschreibung der Schrägung der kegelstumpfförmigen Form des zweiten Reaktionssystemteils des erfindungsgemäßen Reaktionssystems wird der Winkel α verwendet. Der Winkel α ist dabei der zwischen der Mantellinie des Kegelstumpfes und dem Radius der Grundfläche des Kegelstumpfes eingeschlossene Winkel. Die Grundfläche des Kegelstumpfes ist dabei eine angenommene Fläche und existiert in dem Reaktionssystem gemäß der vorliegenden Erfindung nicht als physikalische Fläche, da der erste Reaktionssystemteil und der zweite Reaktionssystemteil bevorzugterweise durchgängig miteinander verbunden sind, so dass das Volumen des ersten Reaktionssystemteils und das Volumen des zweiten Reaktionssystemteils miteinander im Wesentlichen barrierefrei verbunden sind, und ein Flüssigkeitsaustausch zwischen beiden Volumina grundsätzlich frei erfolgen kann. Wie die vorliegenden Erfinder überraschend festgestellt haben, beträgt der Wert des Winkels α bevorzugterweise etwa 55° bis 67°.

Die Größe des erfindungsgemäßen Reaktionssystems ist grundsätzlich weder hinsichtlich der Minimalgröße noch hinsichtlich der Maximalgröße beschränkt, solange eine Kultivierung von photosynthetisch aktiven Zellen darin möglich ist, insbesondere unter den hierin beschriebenen Bedingungen und/oder unter Realisierung der hierin beschriebenen Prozesse und Effekte. Die Größe des erfindungsgemäßen Reaktionssystems in Vertikalrichtung ergibt sich dabei aus der Summe der Höhen - in Vertikalrichtung - der Einzelbestandteile des erfindungsgemäßen - Reaktionssystems; besteht dieses lediglich aus dem ersten Reaktionssystemteil, wobei dieses erste Reaktionssystemteil eine im Wesentlichen zylindrische Form aufweist, und dem zweiten Reaktionssystemteil, wobei dieses zweite Reaktionssystemteil eine im Wesentlichen kegelstumpfförmige Form aufweist, so ergibt sich die Höhe des erfindungsgemäßen Reaktionssystems aus der Summe der Höhe des ersten Reaktionssystemteils, mithin der Höhe des Zylinders, und der Höhe des zweiten Reaktionssystemteils, mithin der Höhe des Kegelstumpfes bzw. der Höhe des Kegels in der Ausführungsform, bei der der zweite Reaktionssystemteil eine kegelförmige Form aufweist. Umfasst das erfindungsgemäße Reaktionssystem noch einen weiteren Teil wie bspw. einen Kopfbereich, so erhöht sich die Höhe des erfindungsgemäßen Reaktionssystems um die Höhe des Kopfbereiches in Vertikalrichtung.

Bei dem erfindungsgemäßen Reaktionssystem weist der erste Reaktionssystemteil eine zylindrische Form auf. Entsprechend weist der erste Reaktionssystemteil eine runde oder kreisförmige Grundfläche auf. Es ist jedoch im Rahmen der vorliegenden Erfindung, dass die Grundfläche des ersten Reaktionssystemteils eine andere als eine kreisförmige Grundfläche aufweist. Eine bevorzugte andere Grundfläche ist dabei eine elliptische Grundfläche, ausgehend von der sich ein Zylinder und damit der erste Reaktionssystemteil erstreckt.

Bei dem erfindungsgemäßen Reaktionssystem weist der zweite Reaktionssystemteil eine kegelstumpfförmige Form auf. Entsprechend weist der zweite Reaktionssystemteil eine runde oder kreisförmige Grundfläche auf. Es ist jedoch im Rahmen der vorliegenden Erfindung, dass die Grundfläche des zweiten Reaktionssystemteils eine andere als eine kreisförmige Grundfläche aufweist. Eine bevorzugte andere Grundfläche ist dabei eine elliptische Grundfläche, ausgehend von der sich ein Kegelstumpf oder, in einer Ausführungsform, ein Kegel, und damit der zweite Reaktionssystemteil erstreckt.

In einer Ausführungsform der vorliegenden Erfindung sind der erste Reaktionssystemteil und der zweite Reaktionssystemteil einstückig ausgebildet, d.h., in Vertikalrichtung, ist der untere Rand des ersten Reaktionssystemteils mit dem oberen Rand des zweiten Reaktionssystemteils verbunden, oder es gehen diese unmittelbar ineinander über. In einer Ausführungsform des erfindungsgemäßen Reaktionssystems ist vorgesehen, dass zwischen dem ersten Reaktionssystemteil und dem zweiten Reaktionssystemteil ein Zwischenbereich angeordnet ist. Bevorzugterweise dient dieser Zwischenbereich dazu, einen Übergang zwischen der Form des ersten Reaktionssystemteils und der Form des zweiten Reaktionssystemteils bereitzustellen, wobei der Übergang insbesondere die Aufrechterhaltung des Strömungsverhaltens der im Reaktionssystem enthaltenen Flüssigkeit gewährleisten soll.

In einer weiteren bevorzugten Ausführungsform des erfindungsgemäßen Reaktionssystems ist der Durchmesser der zylindrischen Form des ersten Reaktionssystemteils, in Vertikalrichtung, über die gesamte Höhe der zylindrischen Form konstant. Es ist jedoch auch im Rahmen der vorliegenden Erfindung, dass der Durchmesser der zylindrischen Form des ersten Reaktionssystemteils, in Vertikalrichtung, über die gesamte Höhe der zylindrischen Form nicht konstant ist. Schließlich ist es im Rahmen der vorliegenden Erfindung, wenn der Durchmesser der zylindrischen Form des ersten Reaktionssystemteils bis kurz vor seinem oberen Ende konstant ist, sich die Form jedoch an seinem oberen Ende ändert; letzteres geschieht vor allem dann, wenn der erste Reaktionssystemteil aus einer Kunststofffolie hergestellt ist und das obere Ende des ersten Reaktionssystemteils durch Verschweißen der Kunststofffolie verschlossen wird. In einer Ausführungsform ist dann vorgesehen, dass sich die Form des ersten Reaktionssystemteils von einer Zylinderform über die oberen 15%, bevorzugterweise über die oberen 10 % und am bevorzugtesten über die oberen 5% der Höhe des Zylinders ändert. Die über diese oberen Höhen von dem ersten Reaktionssystemteil eingenommene Form wird in Wesentlichen durch die Art der Verschweißung bedingt.

In einer weiteren bevorzugten Ausführungsform des erfindungsgemäßen Reaktionssystems ist der Winkel α, in Vertikalrichtung, über die gesamte Höhe des Kegelstumpfes konstant. In einer alternativen Ausführungsform des erfindungsgemäßen Reaktionssystems ist der Winkel α, in Vertikalrichtung, über die gesamte Höhe des Kegelstumpfes nicht konstant.

In der Ausführungsform des erfindungsgemäßen Reaktionssystem, bei der der Durchmesser der zylindrischen Form des ersten Reaktionssystemteils, in Vertikalrichtung, über die gesamte Höhe der zylindrischen Form konstant ist, und bei der der Winkel α der im Wesentlichen kegelstumpfförmigen Form des zweiten Reaktionssystemteils, in Vertikalrichtung, über die gesamte Höhe des als Kegel ausgebildeten Kegelstumpfes konstant ist, kann die Höhe des zweiten Reaktionssystemteils durch tan α berechnet werden.

In einer Ausführungsform des erfindungsgemäßen Reaktionssystems ist vorgesehen, dass dieses eine Begasungseinrichtung umfasst. Bevorzugterweise führt die Begasungseinrichtung ein Gas im Bereich der Deckfläche des Kegelstumpfes in das Reaktionssystem ein, bzw. in der Ausführungsform, in der der zweite Reaktionssystemteil eine kegelförmige Form aufweist, führt die Begasungseinrichtung ein Gas im Bereich der Spitze des Kegels in das erfindungsgemäße Reaktionssystem ein. Die genaue Platzierung der Begasungseinrichtung erfolgt dabei bevorzugterweise so, dass das eingeführte Gas die Durchmischung der in dem erfindungsgemäßen Reaktionssystem enthaltenen Flüssigkeit gewährleistet und/oder das Absetzen, insbesondere das dauerhafte Absetzen, der in der Flüssigkeit enthaltenen photosynthetisch aktiven Zellen verhindert und/oder die an der Mantelfläche des zweiten Reaktionssystemteils nach unten gleitenden photosynthetisch aktiven Zellen wieder in den Flüssigkeitskörper des erfindungsgemäßen Reaktionssystems zurückführt.

Die Begasungseinrichtung ist dabei bevorzugterweise so dimensioniert, dass Volumenströme von 10 ml/min pro Liter in dem Reaktionssystem enthaltener Flüssigkeit bis 45 ml/min pro Liter in dem Reaktionssystem enthaltener Flüssigkeit realisiert werden können. Des Weiteren ist bevorzugt, dass die Begasungseinrichtung grundsätzlich Blasen mit einem Durchmesser von 0,05 cm bis 5 cm erzeugen kann. Die Blasen werden dabei von der Begasungseinrichtung bevorzugterweise als Blasensäule erzeugt.

Das von der Begasungseinrichtung in das erfindungsgemäße Reaktionssystem eingebrachte Gas umfasst bevorzugterweise ein Trägergas und CO₂. Typischerweise macht das Trägergas den größeren Teil des von der Begasungseinrichtung in das Reaktionssystem eingebrachten Volumenstromes aus und ist für die Etablierung und Aufrechterhaltung der Flüssigkeitsströmung in dem erfindungsgemäßen Reaktionssystem verantwortlich. Neben dem Trägergas kann das Gas noch CO₂ enthalten. Dem CO₂-Volumenstrom kommt dabei die Funktion zu, das für die Photosynthese der im Reaktionsgefäß enthaltenen photosynthetisch aktiven Zellen erforderliche CO₂ bereitzustellen, und auch den pH-Wert der im Reaktionssystem enthaltenen Flüssigkeit einzustellen, damit den entsprechenden pH-Erfordernissen der in der Flüssigkeit enthaltenen photosynthetisch aktiven Zellen entsprochen wird. Es wird von den Fachleuten anerkannt werden, dass bei einer Kultivierung der photosynthetisch aktiven Zellen in dem erfindungsgemäßen Reaktionssystem der Anteil des CO₂ am Gesamtvolumenstrom entsprechend variieren und unter bestimmten Bedingungen auch Null sein kann.

Das in das erfindungsgemäße Reaktionssystem eingebrachte Gas verlässt, zumindest zu einem Großteil, das Reaktionssystem wieder. Bevorzugterweise verlässt das Gas aus dem Reaktionssystem am oberen Ende des ersten Reaktionssystemteiles. Dabei ist in einer Ausführungsform der Erfindung vorgesehen, dass das obere Ende des ersten Reaktionssystemteils offen ist. In einer Ausführungsform des erfindungsgemäßen Reaktionssystems ist vorgesehen, dass der erste Reaktionssystemteil an seinem oberen Ende verschlossen ist. Der Verschluss kann dabei durch den oberen Rand oder oberen Bereich des ersten Reaktionssystemteils erfolgen oder dort vorgesehen sein, oder durch einen Kopfbereich erfolgen oder dort vorgesehen sein, wobei sich der Kopfbereich an dem oberen Rand des ersten Reaktionssystemteils anschließt und der Verschluss dann durch den oberen Rand des Kopfbereiches oder den Kopfbereich als Ganzes oder eines Teils davon erfolgt oder dadurch vorgesehen ist. In einer Ausführungsform ist vorgesehen, dass bei einem verschlossenen ersten Reaktionssystemteil, wobei der Verschluss entweder durch den ersten Reaktionssystemteil oder den Kopfbereich bereitgestellt ist, mindestens eine Öffnung im oberen Teil des ersten Reaktionssystemteils oder im Kopfbereich bereitgestellt ist, wobei die Öffnung oberhalb des Flüssigkeitsspiegels im Reaktionssystem angeordnet ist. Die Öffnung kann bspw. in Form eines Loches oder eines Ventils ausgeführt sein. In einer Ausführungsform ist vorgesehen, dass die Öffnung oder die Gesamtheit der Öffnungen so dimensioniert sind, dass im Reaktionssystem bei Begasung ein Überdruck entsteht.

Bei dem erfindungsgemäßen Reaktionssystem handelt es sich bevorzugterweise um einen Photobioreaktor. Entsprechend ist es erforderlich, dass Licht zu den in dem erfindungsgemäßen Reaktionssystem enthaltenen photosynthetisch aktiven Zellen gelangen kann. Gemäß der vorliegenden Erfindung ist vorgesehen, dass zumindest ein Teil des ersten Reaktionssystemteils und/oder ein Teil des zweiten Reaktionssystemteils Eintritt von Licht in das Innere des erfindungsgemäßen Reaktionssystems erlaubt. Ein derartiger Lichteintritt wird bevorzugterweise durch die Auswahl des Materials gewährleistet, aus dem das erfindungsgemäße Reaktionssystem oder Teile davon hergestellt ist. Bevorzugterweise ist das Material ein lichtdurchlässiges Kunststoffmaterial oder Glas.

Das lichtdurchlässige Kunststoffmaterial ist bevorzugterweise ein solches, welches eine hohe Lichtdurchlässigkeit oder aber eine, bevorzugterweise hohe, Lichtdurchlässigkeit für jene Wellenlängen von Licht aufweist, die von den in dem erfindungsgemäßen Reaktionssystem enthaltenen bzw. darin zu kultivierenden photosynthetischen Zellen für ihr Wachstum oder die Produktion eines erwünschten Stoffes benötigt werden. Geeignete Kunststoffe sind im Stand der Technik bekannt. Ein bevorzugter Kunststoff ist ein Polyethylen-Schlauch, wobei dieser bevorzugterweise leicht dehnbar, transparent und eine hohe Lichtdurchlässigkeit aufweist. Eine weitere vorteilhafte Eigenschaft, die ein Kunststoff aufweist, der in einer Ausführungsform des erfindungsgemäßen Reaktionssystems Verwendung findet für die Ausbildung der ersten und/oder zweiten Reaktionssystemteils, ist seine gute Schweißbarkeit.

Die gute Schweißbarkeit des Kunststoffes erlaubt, insbesondere ausgehend von einem Kunststofffolienschlauch, eine einfache Ausbildung des ersten Reaktionssystemteils und des zweiten Reaktionssystemteils, wobei das diese umfassende erfindungsgemäße Reaktionssystem bevorzugterweise einstückig ausgebildet ist. Die Ausbildung der kegelstumpfförmigen Form des zweiten Reaktionssystemteils kann konkret dadurch erfolgen, dass am unteren Ende des ersten Reaktionssystemteils ein Kegelstumpf und damit das zweite Reaktionssystemteil ausgebildet wird, indem der Schlauch beginnend mit dem unteren Rand des den ersten Reaktionssystemteil ausbildenden Schlauchabschnittes nach unten unter Beibehaltung des erwünschten Winkels α verschweißt wird.

Die Elastizität des Kunststoffmaterials erlaubt in einer Ausführungsform des erfindungsgemäßen Reaktionssystems, dass Einlässe und Auslässe durch Einführen entsprechender Nadeln bereitgestellt werden, da sich das Kunststoffmaterial dichtend um die Einstichstelle schließt.

Hinsichtlich der Art der in dem erfindungsgemäßen Reaktionssystem zu kultivierenden photosynthetisch aktiven Zellen bestehen grundsätzlich keine Einschränkungen. Beispielhaft seien hier die Gattungen Chlorella, Nannochloropsis, Scenedescmus, Porphyridium, Spirulina, Chlamydomonas und Oedogonium genannt.

Es ist im Rahmen der vorliegenden Erfindung, dass das erfindungsmäße Reaktionssystem noch weitere Einrichtungen umfasst, wie sie bei Reaktionssystemen für die Kultivierung photosynthetisch aktiver Zellen und insbesondere Photobioreaktoren nach dem Stand der Technik üblich sind.

In einem weiteren Aspekt betrifft die vorliegende Erfindung auch eine Reaktionssystemanordnung, welche mindestens zwei der erfindungsgemäßen Reaktionssysteme, bevorzugterweise eine Vielzahl der erfindungsgemäßen Reaktionssysteme umfasst. In einer Ausführungsform ist dabei vorgesehen, dass die mindestens zwei erfindungsgemäßen Reaktionssysteme parallel zu einander betrieben werden. In einer derartigen Parallelschaltung ist bevorzugterweise vorgesehen, dass ausgehend von einer zentralen Versorgungsleitung die Gasversorgung der mindestens zwei erfindungsgemäßen Reaktionssysteme parallel erfolgt und/oder eine die jeweilige mindestens eine Ernteöffnungen der mindestens zwei erfindungsgemäßen Reaktionssystems vereinigende Ernteleitung vorhanden ist. Grundsätzlich können bei einer derartigen parallelen Anordnung von mindestens zwei der erfindungsgemäßen Reaktionssysteme auch weitere Einrichtungen vorhanden sein, die von den parallel angeordneten erfindungsgemäßen Reaktionssystemen gemeinsam genutzt werden.

In einem noch weiteren Aspekt betrifft die vorliegende Erfindung ein Verfahren zur Kultivierung von photosynthetisch aktiven Zellen, wobei das Verfahren Kultivieren von photosynthetisch aktiven Zellen in einem erfindungsgemäßen Reaktionssystem umfasst.

Es ist im Rahmen der vorliegenden Erfindung, dass in dem erfindungsgemäßen Verfahren grundsätzlich eine jede Art von photosynthetisch aktiven Zellen kultiviert werden kann. Die Erfordernisse für die Kultivierung derartiger photosynthetisch aktiver Zellen sind den Fachleuten auf dem Gebiet bekannt oder können im Rahmen von Routineuntersuchungen bestimmt werden. Die Erfordernisse umfassen insbesondere die Art und Zusammensetzung des für die Kultivierung erforderlichen Mediums, welches in einer Ausführungsform hierin auch als Flüssigkeit in dem erfindungsgemäßen Reaktionssystem bezeichnet wird, die erforderliche Lichtquantität und Lichtqualität, den erforderlichen pH-Wert und die erforderliche Temperatur.

In einer Ausführungsform ist vorgesehen, dass das erfindungsgemäße Verfahren in einem Gewächshaus durchgeführt wird. Dabei ist bevorzugterweise vorgesehen, dass keine künstlichen Lichtquellen vorgesehen sind und/oder verwendet werden; entsprechend steht für die erfindungsgemäße Kultivierung der photosynthetisch aktiven Zellen nur das in das Gewächshaus eindringende Sonnenlicht zur Verfügung. Des Weiteren ist bei der Ausführungsform des erfindungsgemäßen Verfahrens, das in einem Gewächshaus durchgeführt wird, in einer bevorzugten Ausführungsform vorgesehen, dass keine zusätzliche Beheizung des erfindungsgemäßen Reaktionssystems erfolgt; entsprechend entspricht die Temperatur in dem erfindungsgemäßen Reaktionssystem im Wesentlichen der Temperatur des Gewächshauses. Die Temperatur des Gewächshauses kann entweder dem Tagesgang der Temperatur entsprechen, wie er am Standort des Gewächshauses vorliegt; die Temperatur des Gewächshauses kann aber auch durch Heizen und/oder Kühlen zumindest bis zu einem gewissen Grad vom Tagesgang der Temperatur unabhängig gestaltet werden, wie er am Standort des Gewächshauses vorliegt.

Das erfindungsgemäße Verfahren kann grundsätzlich in allen im Stand der Technik bekannten Betriebsarten durchgeführt werden, insbesondere im batch, repeated batch und kontinuierlichem Betrieb. Es wird von den Fachleuten anerkannt werden, dass das erfindungsgemäße Reaktionssystem mit weiteren Einrichtungen versehen werden kann oder muss, um diese verschiedenen Betriebsarten zu ermöglichen.

Die vorliegende Erfindung wird nun an Hand der folgenden Figuren weiter beschrieben, aus denen sich weitere Merkmale, Ausführungsformen und Vorteile derselben ergeben. Dabei zeigt
Fig. 1 eine schematische Darstellung eines Querschnittes durch eine Ausführungsform des erfindungsgemäßen Reaktionssystems;
Fig. 2 eine schematische Darstellung eines Teils der in Fig. 1 gezeigten Ausführungsform des erfindungsgemäßen Reaktionssystems zusammen mit einer Begasungseinrichtung;
Fig. 3 eine schematische Darstellung eines Querschnittes durch eine weitere Ausführungsform des erfindungsgemäßen Reaktionssystems; und
Fig. 4 eine schematische Darstellung eines Teils der in Fig. 2 gezeigten Ausführungsform des erfindungsgemäßen Reaktionssystems zusammen mit einer Begasungseinrichtung.

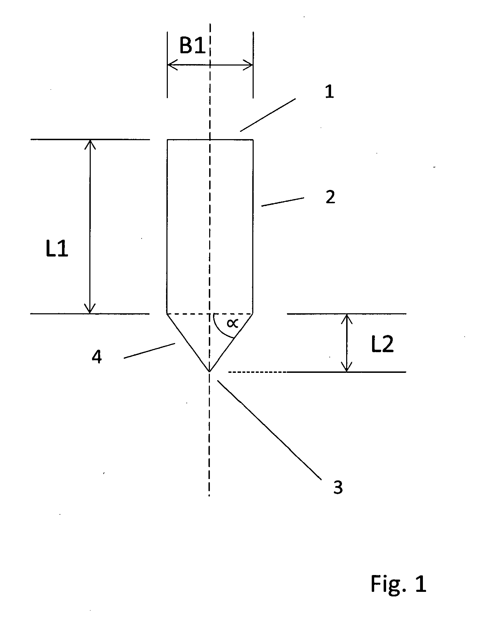

Fig. 1 zeigt eine schematische Darstellung eines Querschnittes durch eine Ausführungsform des erfindungsgemäßen Reaktionssystems. In der dargestellten Ausführungsform des erfindungsgemäßen Reaktionssystems weist der erste Reaktionssystemteil eine zylindrische Form auf. Der erste Reaktionssystemteil ist in Fig. 1 durch das durch die Höhe L1 und die Breite B1 definierte Rechteck dargestellt und umfasst einen oberen Rand 1 und einen seitlichen Rand 2. Der obere Rand 1 begrenzt den ersten Reaktionssystemteil noch oben und schließt diesen gegenüber der Umwelt ab; der seitliche Rand 2 begrenzt den ersten Reaktionssystemteil zur Seite und schließt diesen gegenüber der Umwelt ab. Der Durchmesser des Zylinders ist durch die Breite B1 definiert.

In der in Fig. 1 dargestellten Ausführungsform des erfindungsgemäßen Reaktionssystems weist der zweite Reaktionssystemteil eine kegelförmige Form auf. Der zweite Reaktionssystemteil ist in Fig. 1 durch das durch die Höhe L2 definierte Dreieck dargestellt und umfasst eine Spitze 3. Die - gedachte - Grundfläche des Kegels ist durch die gestrichelte horizontale Linie dargestellt. Der Kegel weist eine Mantelfläche 4 auf, die als Gleitfläche für die im Reaktionssystem nach unten sinkenden Zellen dient und diese zu der Spitze 3 leitet, von wo aus sie wieder in den Flüssigkeitskörper des erfindungsgemäßen Reaktionssystem eingebracht werden. Die Vertikalachse ist in Fig. 1 als gestrichelte Linie dargestellt.

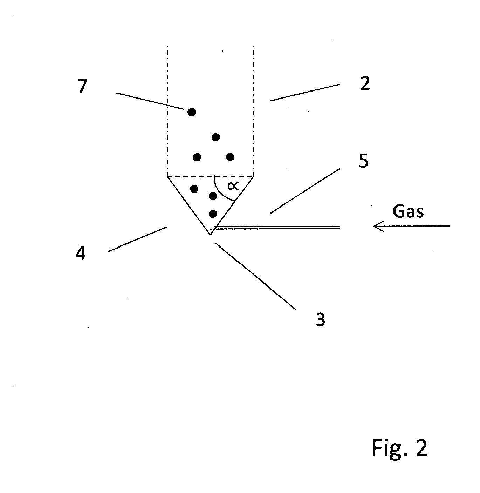

Fig. 2 zeigt eine schematische Darstellung eines Teils der in Fig. 1 gezeigten Ausführungsform des erfindungsgemäßen Reaktionssystems zusammen mit einer Begasungseinrichtung 5. Die Begasungseinrichtung 5 ist schematisch als Rohr dargestellt, durch welches Gas in den zweiten Reaktionssystemteil eingeführt wird. Die Begasungseinrichtung ist dabei so in den zweiten Reaktionssystemteil eingeführt, dass deren Gasauslassöffnung in etwa in der Mitte des Kegels nahe an der Kegelspitze 3 angeordnet ist. Das aus der Gasauslassöffnung austretende Gas tritt in Form von einzelnen Blasen 7 aus. Der teilweise dargestellt seitliche Rand 2 des ersten Reaktionssystemteils ist schematisch als gestrichelte Linie dargestellt.

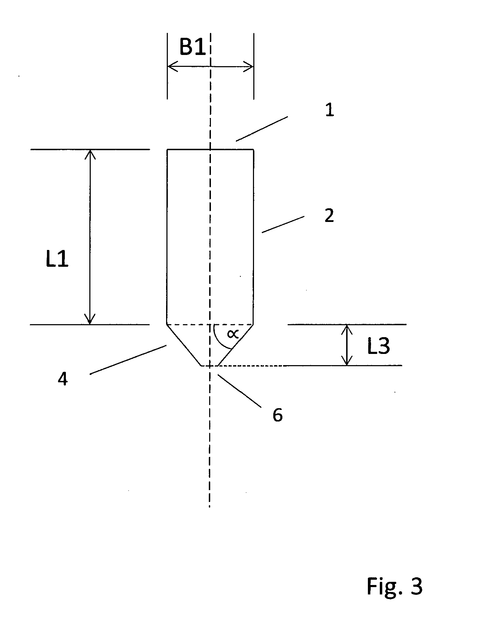

Fig. 3 zeigt eine schematische Darstellung eines Querschnittes durch eine weitere Ausführungsform des erfindungsgemäßen Reaktionssystems.

Die in Fig. 3 gezeigte Ausführungsform des erfindungsgemäßen Reaktionssystems entspricht in ihrem grundsätzlichen Aufbau dem der in Fig. 1 gezeigten Ausführungsform, wobei der zweite Reaktionssystemteil eine kegelstumpfförmige Form aufweist. Der Kegelstumpf weist eine Höhe L3 und eine Deckfläche 6 auf, die die untere Begrenzung des zweiten Reaktionssystemteils und damit des Reaktionssystems insgesamt darstellt.

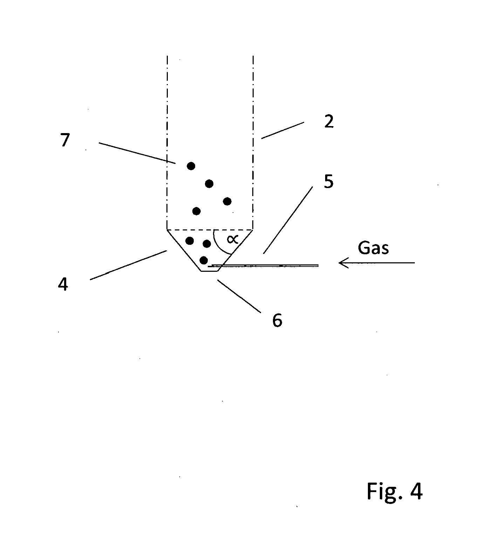

Fig. 4 zeigt eine schematische Darstellung eines Teils der in Fig. 3 gezeigten Ausführungsform des erfindungsgemäßen Reaktionssystems zusammen mit einer Begasungseinrichtung 5. Die Begasungseinrichtung 5 ist identisch zu der in Fig. 2 dargestellten Ausführungsform des erfindungsgemäßen Reaktionssystems ausgeführt, wobei bei der in Fig. 4 dargestellten Ausführungsform die Begasungseinrichtung so in den zweiten Reaktionssystemteil eingeführt ist, dass deren Gasauslassöffnung in etwa in der Mitte des Kegelstumpfes nahe an der Deckfläche 6 angeordnet ist.

Die in der vorstehenden Beschreibung, den Ansprüchen sowie den, Zeichnungen offenbarten Merkmale der Erfindung können sowohl einzeln als auch in beliebigen Kombinationen für die Verwirklichung der Erfindung in ihren verschiedenen Ausführungsformen wesentlich sein.

### Bezugszeichenliste

- 1: oberer Rand
- 2: seitlicher Rand
- 3: Kegelspitze
- 4: Mantelfläche
- 5: Begasungseinrichtung
- 6: Kegelstumpfdeckfläche
- 7: Gasblase
- B1: Breite des ersten Reaktionssystemteils
- L1: Höhe des ersten Reaktionssystemteils
- L2: Höhe des Kegels
- L3: Höhe des Kegelstumpfes

## Patentansprüche

1. Reaktionssystem geeignet für die Kultivierung von photosynthetisch aktiven Zellen, umfassend in vertikaler Anordnung
- einen ersten Reaktionssystemteil, wobei der erste Reaktionssystemteil eine im Wesentlichen zylindrische Form aufweist, und
- einen zweiten Reaktionssystemteil, wobei der zweite Reaktionssystemteil eine im Wesentlichen kegelstumpfförmige Form aufweist,
wobei zumindest ein Teil der ersten Reaktionssystemteils und/oder ein Teil des zweiten Reaktionssystemteils Eintritt von Licht in das Innere des Reaktionssystems erlaubt und wobei die kegelstumpfförmige Form des zweiten Reaktionssystemteils einen Winkel α aufweist, der Winkel α der zwischen der Mantellinie des Kegelstumpfes und dem Radius der Grundfläche des Kegelstumpfes eingeschlossene Winkel ist, und der Winkel α einen Wert von 55° bis 67° aufweist.

2. Reaktionssystem nach Anspruch 1, wobei der Winkel α einen Wert von 60° bis 65° aufweist, bevorzugterweise einen Wert von 65°aufweist.

3. Reaktionssystem nach einem der Ansprüche 1 bis 2, wobei der erste Reaktionssystemteil in Vertikalrichtung eine Höhe von 50 cm bis 135 cm aufweist. bevorzugtererweise der erste Reaktionssystemteil in Vertikalrichtung eine Höhe von 120 cm bis 135 cm aufweist und am bevorzugtesten eine Höhe von 125 cm bis 130 cm aufweist.

4. Reaktionssystem nach einem der Ansprüche 1 bis 3, wobei der Radius der zylindrischen Form des ersten Reaktionssystemteils einen Wert von 7,5 cm bis 40 cm aufweist, bevorzugterweise einen Wert von 10 cm bis 17,5 cm aufweist und am bevorzugtesten einen Wert von 15 cm bis 16 cm aufweist.

5. Reaktionssystem nach einem der Ansprüche 1 bis 4, wobei die Höhe des ersten Reaktionssystemteils in Vertikalrichtung 130 cm beträgt, der Radius der zylindrischen Form des ersten Reaktionssystemteils 15,5 cm beträgt und die kegelstumpfförmige Form des zweiten Reaktionssystemteils ein Kegel ist, wobei die Höhe der kegelstumpfförmigen Form des zweiten Reaktionssystemteils 34 cm beträgt.

6. Reaktionssystem nach einem der Ansprüche 1 bis 5, wobei das Reaktionssystem eine Begasungseinrichtung zum Einführen von Gas in das Reaktionssystem umfasst, wobei die Begasungseinrichtung das Gas im Bereich der Deckfläche des zweiten Reaktionssystemteils mit im Wesentlichen kegelstumpfförmiger Form einführt.

7. Reaktionssystem nach Anspruch 6, wobei die Begasungseinrichtung eine solche ist, die eine Blasensäule erzeugen kann, wobei die Blasen der Blasensäule einen Durchmesser von 0,05 cm bis 5 cm aufweisen, bevorzugterweise einen Durchmesser von 0,2 cm bis 3 cm aufweisen und am bevorzugtesten einen Durchmesser von 0,5 cm bis 2 cm aufweisen.

8. Reaktionssystem nach einem der Ansprüche 6 bis 7, wobei ein durch die Begasungseinrichtung in das Reaktionssystem eingebrachter Volumenstrom 10 ml/min pro Liter in dem Reaktionssystem enthaltener Flüssigkeit bis 45 ml/min pro Liter in dem Reaktionssystem enthaltener Flüssigkeit beträgt, bevorzugterweise ein durch die Begasungseinrichtung in das Reaktionssystem eingebrachter Volumenstrom 12 ml/min pro Liter in dem Reaktionssystem enthaltener Flüssigkeit bis 20 ml/min pro Liter in dem Reaktionssystem enthaltener Flüssigkeit beträgt und am bevorzugtesten ein durch die Begasungseinrichtung in das Reaktionssystem eingebrachter Volumenstrom 13 ml/min pro Liter in dem Reaktionssystem enthaltener Flüssigkeit bis 16 ml/min pro Liter in dem Reaktionssystem enthaltener Flüssigkeit beträgt.

9. Reaktionssystem nach einem der Ansprüche 1 bis 8, wobei der erste Reaktionssystemteil und der zweite Reaktionssystemteil jeweils und unabhängig aus einem Material besteht, das ausgewählt ist aus der Gruppe umfassend lichtdurchlässige Kunststofffolie und Glas.

10. Reaktionssystem nach einem der Ansprüche 1 bis 9, wobei der erste Reaktionssystemteil und der zweite Reaktionssystemteil zusammen einstückig und aus einer lichtdurchlässigen Folie ausgebildet sind.

11. Reaktionssystem nach einem der Ansprüche 1 bis 10, wobei die photosynthetisch aktiven Zellen ausgewählt sind aus der Gruppe umfassend Pflanzenzellen und Algenzellen, bevorzugterweise sind die photosynthetisch aktiven Zellen ausgewählt aus der Gruppe umfassend Chlorella, Nannochloropsis, Scenedescmus, Porphyridium, Spirulina, Chlamydomonas und Oedogonium.

12. Reaktionssystem nach einem der Ansprüche 1 bis 11, wobei das Reaktionssystem photosynthetisch aktive Zellen enthält und wobei die photosynthetisch aktiven Zellen ausgewählt sind aus der Gruppe umfassend Pflanzenzellen und Algenzellen, bevorzugterweise sind die photosynthetisch aktiven Zellen ausgewählt aus der Gruppe umfassend Chlorella, Nannochloropsis, Scenedescmus, Porphyridium, Spirulina, Chlamydomonas und Oedogonium.

13. Reaktionssystemanordnung umfassend mindestens zwei Reaktionssysteme nach einem der Ansprüche 1 bis 12.

14. Verfahren zur Kultivierung von photosynthetisch aktiven Zellen umfassend.
- Kultivieren von photosynthetisch aktiven Zellen in einem Reaktionssystem nach einem der Ansprüche 1 bis 12 oder einer Reaktionssystemanordnung nach Anspruch 13.

15. Verfahren nach Anspruch 14, wobei sich das Reaktionssystem oder die Reaktionssystemanordnung in einem Gewächshaus befindet, die Temperierung des Reaktionssystems oder der Reaktionssystemanordnung über eine Temperierung des Gewächshauses erfolgt und/oder das für die Photosynthese der photosynthetisch aktiven Zellen erforderliche Licht durch Sonnenlicht und nicht durch eine künstliche Lichtquelle bereitgestellt wird.
